# EUROPEAN PATENT SPECIFICATION

(11) **EP 0 702 665 B1**
(45) Date of publication and mention of the grant of the patent: **09.08.2000**
(21) Application number: 94916049.3
(22) Date of filing: 06.05.1994
(51) Int. Cl.: C07C 11/12, C07C 2/04

(54) **PROCESS FOR PRODUCING 1,4-DIENES**
VERFAHREN ZUR HERSTELLUNG VON 1,4-DIENEN
PROCEDE DE PRODUCTION DE 1,4-DIENES

(30) Priority: 06.05.1993 US 58616
(43) Date of publication of application: 27.03.1996
(73) Proprietor: EXXON CHEMICAL PATENTS INC., Baytown, TX 77520-2149 (US)
(72) Inventor: HENDRIKSEN, Dan, Eldon, Kingwood, TX 77345 (US); LINSCOTT, Stephanie, Austin, TX 78705-3822 (US)
(74) Representative: Dew, Melvyn John
(86) International application number: PCT/US94/05095
(87) International publication number: WO 94/26682

(56) References cited:
- DE-A- 1 593 574
- US-A- 3 405 194
- CHEMICAL ABSTRACTS, vol. 103, no. 15, 14 October 1985, Columbus, Ohio, US; abstract no. 122975n, page 682 ; & JP,A,60 089 436 (MITSUBISHI PETROCHEMICAL CO) 20 May 1985

## Description

This invention relates to processes for the preparation of 1,4-dienes from the reaction of a conjugated diolefin with an alpha-monoolefin, and more particularly, to such processes involving a catalyst system including a salt of cobalt, a tertiary diphosphine, and an organoaluminum compound.

1,4-Dienes are useful as comonomers in polyolefin polymers since they allow the polymer to be cross linked.

US-A-3,405,194 teaches a method of producing hexadienes by the reaction of an alpha-monoolefin with a conjugated diolefin in the presence of a catalyst system of a salt or cobalt or iron, a tertiary diphosphine, and an organoaluminum compound. The tertiary diphosphines include 1,2-bis (diphenylphosphino) ethane ("dppe") and 1,3 -bis (diphenylphosphino) propane ("dppp").

The examples, and certain contemporaneous and subsequent publications, teach the importance of reacting a conjugated diolefin with an alpha-monoolefin at temperatures of 80° C or more when using a cobalt based catalyst system of the types described. Thus of the 88 examples in the patent, 68 examples (1-53 and 74-88) are directed to a catalyst system of a salt of cobalt, a tertiary diphosphine, and an organoaluminum compound, and 64 of these 68 examples employ a temperature of at least 80° C (example 2 is at 70° C and examples 39, 47 and 76 are at 50° C, all with a catalyst system including a cobalt complex with dppe).

In "A New Process for 1,4-Diene Synthesis", A. Miyake, G. Hata, M. Iwamoto, and S. Yuguchi, *Proceedings of the Seventh World Petroleum Congress*, 1967 volume 5, 317, the authors described the superiority of a cobalt diphosphine system of CoC1₂-dppe in combination with an aluminum trialkyl, specifically, triethylaluminum, at 80° C (each also described in US-A-3,405,194).

The effects of reaction temperature on reaction rate for butadiene conversion are particularly described for the catalyst system of CoC1₂-dppe-Et3A1 in "Reaction of Butadiene with Ethylene. IV. Synthesis of 1,4-Hexadiene by a Cobalt Choride-Ditertiary Phosphine Complex and an Organolaluminum Compound Catalyst", M. Iwamoto and S. Yuguchi, *Bulletin of the Chemical Society of Japan*, 1968, 41, 150. Apparent optimum reaction temperatures are said to be in the range of 80 to 100° C. Two examples achieved 97% selectivity at 80° C. Below 80° C, rate of conversion is shown to fall precipitously, and at temperatures higher than 100° C a considerable loss of selectivity from isomerization of 1,4 hexadiene to conjugated dienes, mainly to 2,4-hexadiene, is reported.

Henrici-Oliv et al. in "Codimerization of Butadiene and Ethylene," *Journal of Organometallic Chemistry*, vol. 35, p. 381 (1972), a study of the catalytic system described in the above cited articles by Iwamoto et al. report that temperature of reaction appears critical for high selectivity using CoC1₂-dppe-Et₃A1 in dichloroethane. Page 381 expressly states that "between 80° C and 110° C *cis*-1,4-hexadiene is formed in high yield, whereas below 80° C more ethylene than butadiene is consumed with the result that C₈ compounds are produced. Above 110° C 1,4-hexadiene is isomerized to 2,4-hexadiene." Teaching the same is US-A-3,647,902, to Henrici-Oliv et al. (where the catalyst system is CoC1₂-dppe-Et₃A1 in a halogenated hydrocarbon solvent), stating that the temperature must be not less than 80° C when ethylene is reacted with 1,3-butadiene, or poor selectivity results (col. 2, lines 13-24).

The foregoing teachings in the prior art on conducting the reaction at temperatures of at least 80° C encompass specific described cobalt catalyst systems where the diphosphine ligand is dppp. In US-A-3,405,194, examples 18-27, 43 and 88 employ dppp as a cobalt catalyst ligand. In examples 18-27, the cobalt complex is CoC1₂ and dppp; in examples 43 and 48, the cobalt complex is the acetyl acetonate of trivalent cobalt, i.e., Co(C₅H₇O₂)₃, with the dppp ligand. (Acetylacetonate is sometimes referred to as "acac"; for example, the acetylacetonate of trivalent cobalt is Co(acac)₃). The organoaluminum compound used in examples 18-27, 43 and 88 is trimethylaluminum ("TMAL") or triethylaluminum ("TEAL") when the cobalt salt is CoC1₂ and is sometimes diethylaluminum chloride ("DEAC") when the cobalt salt is Co(acac)₃. In all these examples employing the dppp ligand, the temperatures are in the range 80° - 102° C.

"Effects of Ditertiary Phosphine Ligands in Co-dimerization of Butadiene and Ethylene Catalyzed by cobalt Chloride-Ditertiary Phosphine-Triethylaluminum" T. Kagawa, Y. Inoue, and H. Hashimoto, *Bulletin of the Chemical Society of Japan*, 1970, 43, 1250, reports results for testing a CoC1₂-Ph₂P(CH₂)ₙPPh₂Et₃A1 catalyst system (Ph = phenyl, n = 1,2,3,4 etc.) for effect of length of the methylene chain between the two phosphorus atoms on *cis*-1,4-hexadiene formation at 80-90° C. The data presented for the system CoC1₂-dppp-Et₃A1 indicate a relatively low yield to 1,4-hexadiene on butadiene.

The temperature teachings in the art have applied whether the conjugated diolefin is butadiene or isoprene. Examples 74-75 and 77-82 of US-A-3,405,194 employ CoC1₂-dppe-Et₃A1 at 80 °C or 100° C to react isoprene with ethylene. In "Preparation of 3-Methyl-1,4-heptadiene, 3-Ethyl-1,4-hexadiene, and Methyl-1,4-hexadienes" by M. Iwamoto, K. Tani, H. Igaki, and S. Yuguchi, *Journal of Organic Chemistry* 1967, 32, 4148, reaction of isoprene with ethylene is reported rapid and selective (97.2 - 99%) at 80-98° C with the CoC1₂-dppe-Et₃A1 catalyst system.

Even variants of the cobalt catalyst systems teach reaction conversion of conjugated diolefins to 1,4-dienes at a 80° C minimum. US-A-3,445,540 uses a triphosphine instead of a diphosphine, and US-A-3,574,139 uses cobalt complexed with two diphosphines, i.e., bis [ethylene bis (diphenylphosphine)] cobalt (0) or bis [ethylene bis (diphenylphosphine)] cobalt (I) hydride. Both of these two patents state that "temperatures below 80° C may be too slow for operating convenience. The preferred temperature range lies between 80 and 120° C..." (US-A-3,574,139, col. 3, lines 70-74; US-A-3,445,540, col. 5, lines 35-39.)

In US-A-3,405,194, toluene is the solvent in examples 18-23 and 43; halogenated hydrocarbon solvents are used in examples 24-27 and 88. Halogenated hydrocarbon solvents are instructed to be used to improve selectivity when the intended hexadiene is 1,4-hexadiene, and to provide the further advantage of easier cobalt catalyst handling, since these solvents dissolve the cobalt complex completely (col. 4, lines 60-67). In the article by Henrici-Oliv et al. cited above, halogenated hydrocarbon solvents are said to be favored, because CoC1₂-dppe is insoluble in toluene and alkylation reactions of transition metal salts with aluminum alkyls are faster and more complete in halogenated solvents than in aromatics. US-A-3,647-902 contain a similar teaching

According to the present invention there is provided a process for producing substantially pure 1,4 dienes by converting a conjugated diolefin to the 1,4-diene with substantially 100 % selectivity, which comprises reacting a conjugated diolefin with an alpha-olefin in a hydrocarbon solvent reaction medium and in the presence of a catalyst system consisting essentially of a cobalt compound, a tertiary diphosphine and an organo aluminum compound, characterized in that the catalyst system comprises:
a) Co(acac)ₙ wherein from 0.9 to less than 2 mols of dppp are combined with the Co(acac)ₙ, or a preformed complex of CoCl₂(dppp), where acac is acetylacetonate, dppp is 1,3-bis (diphenylphosphino)propane, and n is the valence of cobalt; and
b) a dialkylaluminum chloride,
wherein the catalyst system has an Al/Co molar ratio of at least 5:1, the reaction is performed at a temperature of at least 50°C and less than 70°C and at an alpha-olefin partial pressure of from 50 to 10,000 kPa (0.5 to 100 atmospheres)

By "pure" is generally meant more than about 99% 1,4-diene, preferably approaching or equaling 100% 1,4-diene.

The highest selectivity, essentially 100 percent selectivity, is desired, in order to maximize production of pure 1,4-dienes, thereby eliminating the difficult separation of 2,4-diene and other by-products to satisfy the commercial need for pure 14-dienes. It has been found that substantially 100% selectivity from reaction of conjugated diolefins with an alpha-olefin is possible using the process according to the invention. Preferably the selectivity is greater than 98.5%, more preferably greater than 99%, most preferably 99.5% and ideally 100%. It is also possible by selection of reaction conditions within the specified ranges to provide essentially pure 1,4-dienes from conjugated diolefins at high rates of reaction and/or high catalyst productivity.

In order to obtain highest reaction rates per unit of catalyst, the prior art has employed halogenated hydrocarbon solvents for preformed catalysts, particularly the favored CoC1₂-dppe/TEAL catalyst system of the above referenced prior an. However, halogenated solvents are environmentally disfavored, are corrosive to equipment, and consequently are commercially unattractive as a means of improving catalytic reaction rates and product yields. With the process of this invention it is possible to promote reaction of conjugated diolefins and alpha-olefins at high rates of reaction without the use of halogenated hydrocarbon solvents.

Surprisingly, contrary to the teachings of the prior art, such results are achievable at temperatures less than 80° C. Further, exceptional selectivity at these reaction temperatures in this particular catalyst system may be accompanied by vastly increased rates of reaction and catalyst productivity, indicative of a very reactive species of catalyst. Still more surprisingly, these results may be accomplished without use of the corrosive and environmentally disfavored halogenated hydrocarbon solvents that the prior art teaches should be used to improve yields.

The catalysts employed in the process of this invention are Co(acac)ₙ combined with dppp and preformed CoC1₂(dppp), and the cocatalysts are selected from dialkylaluminum chloride of the general formula R' R'' AlX, where R is an alkyl, suitably in the C₁ - C₄ range, Al is aluminum, and X signifies chloride, for example, dimethylaluminum chloride, diethylaluminum chloride, di-n-propylaluminum chloride, di-isopropylaluminum chloride, and di-isobutylaluminum chloride. Conjugated diolefins employed suitably are 1,3-butadiene (sometimes referred to herein simply as butadiene or "BD") or isoprene, and the alpha monoolefins suitably are ethylene or propylene. Where the reactants are butadiene and ethylene, pure 1,4-hexadiene is produced.

When the cobalt catalyst is Co(acac)ₙ, 0.9 to less than 2 mols of dppp are combined with the Co(acac)ₙ in the reaction medium. Advantageously, the mol ratio of dppp to Co(acac)ₙ is effective to produce a rate of reaction, at a reaction temperature less than 70° C, preferably around 60° C, of at least about 120 mols of 1,4-hexadiene per mol of cobalt per minute, where the conjugated diene is butadiene or where isoprene or propylene are the reactants, of at least 30 mols of pure product per mol of cobalt per minute. Advantageously, the mol ratio of dppp to Co(acac)ₙ is effective for a catalyst productivity, at the reaction temperature less than 70° C, of at least about 1000 mols of 1,4-hexadiene per mol of cobalt.

In a preferred embodiment, the catalyst system is Co(acac)₂ combined with dppp and diethylaluminum chloride or Co(acac)₃ combined with dppp and diethylaluminum chloride.

In another preferred embodiment, the process catalyst system is CoC1₂(dppp) and diethylaluminum chloride. Using this system in the process, the rate of the reaction suitably exceeds 100 mols of 1,4-hexadiene produced per mol of cobalt per minute. The most preferred embodiment is a process for preparing pure 1,4-hexadiene, which comprises reacting butadiene with ethylene in the presence of a catalyst system consisting of (a) Co (II) (acetylacetonate)₂ and 1,3- bis (diphenylphosphino) propane in a preferred mol ratio of about 1:1.5, and (b) diethylaluminum chloride, at a mol ratio of A1/Co of at least 5:1, in a hydrocarbon solvent at reaction conditions controlled in a temperature range from about 50° C to 69° C and an alpha-olefin partial pressure range from 50 to 10,000 kPa (.5 to 100 atmospheres), effective to convert the butadiene to 1,4-hexadiene with essentially 100% selectivity at a reaction rate of at least 500 mols of 1,4-hexadiene produced per mol of cobalt per minute.

Diethylaluminum chloride as cocatalyst is used alone when the catalyst is formed in situ from Co(acac)₃ or Co(acac)₂. When the improved preformed complex CoC1₂(dppp) is used, triethylaluminum, preferably up to two equivalents (Et₃A1:Co not more than 2), may be added with the diethylaluminum chloride.

The temperature of the reaction has a dramatic effect on the selectivity of the reaction to the desired 1,4-diene with the catalyst systems employed in the process of the present invention. The process temperature is from 50° C to less than 70° C, and most preferably horn 55° C to 65° C, eg about 60° C. In examples 1 through 3 and 5 through 10 below of the present improved process, the temperature of reaction is 60° C. Selectivity to 1,4-hexadiene in these Examples approaches 100%. The reaction rate and catalyst productivity are very high with the improved catalyst despite the improvement in selectivity by use of a lower temperature.

The improved process may use a hydrocarbon solvent such as toluene or xylene or the 1,4-diene product of the reaction, e.g. 1,4-hexadiene from the reaction of butadiene with ethylene. No separation of a solvent from the product is needed when the product is used as the solvent for the reaction. Halogenated solvents are usable but unnecessary for adequate results.

The present invention is illustrated by the following Examples 1 through 3 and 5 through 10. In all of these examples except Example 5, data is given on (1) conversion of the charged conjugated diene by a catalyst system, i.e., the percent conversion of a conjugated diene charge to all products of reaction, (2) productivity of a catalyst system. i.e., yield of the desired 1,4-diene product per mol of cobalt catalyst, (3) selectivity of the catalyst system for the desired 1,4-diene, i.e., the percent of all the products which is the desired 1,4-diene, (4) the observed first order rate constant of the catalyst system, and (5) the overall reaction rate of the catalyst system.

Thus, "conversion" of diene (expressed as a percentage) is mols of diene converted to all products divided by mols of diene charged (for 1,3-butadiene, percent conversion is the percent of mol 1,3-butadiene converted to all products/mol 1,3-butadiene charged). "Selectivity" (expressed as percentage) is the mols yield of a particular diene product divided by the mols of diene converted to all products. Thus, selectivity of the catalyst system for 1,4-hexadiene with a charge of ethylene and 1,3-butadiene is mols of 1,4-hexadiene yield/mols 1,3-butadiene convened to all products. "Yield" of a desired product results from multiplying the selectivity percentage of the catalyst system for that desired product under the conditions of the reaction times the aggregate mols of all the products into which the charge diene is converted.

The first order rate constant in the Examples is helpful for within system comparisons. As is shown, the first order reaction rate constant per mmole of cobalt can be about 15. Ethylene pressure in a 500 mL vessel was recorded vs. time during the reaction as the ethylene was fed to the reactor to keep the pressure constant. The reaction was permitted to proceed for a long enough time to determine the pressure of ethylene remaining after all butadiene had been consumed. The Pₜᵢₘₑ-P_{final} is then proportional to the amount of butadiene remaining in the autoclave. The reaction is first order for butadiene, and a plot of ln(Pₜᵢₘₑ-P_{final}) vs. time yields a straight line, the slope of which is the observed rate constant.

### Example 1

In a nitrogen-filled glove box 6 mg (0.01 mmole) of a preformed complex of CoC1₂ (1,3-bis (diphenylphosphino) propane) was dissolved in 80 mL anhydrous toluene. To this was added with stirring a 25 wt% solution of diethylaluminum chloride in toluene (0.6 mL, 1.0 mmole). This solution was transferred under nitrogen to a 300 mL stirred autoclave and heated to 60° C. With the stirrer stopped, butadiene (20.8 g, dried and with *t*-butylcatechol inhibitor removed by passing through a column of 3A molecular sieve and a column of basic alumina) was pressured into the autoclave using ethylene and the total pressure brought to 2089 kPa with the ethylene. The stirrer was started, and the total pressure in the autoclave was kept constant at 1400 kPa (200 psig) by feeding ethylene through a pressure regulator from a 500 mL vessel containing ethylene at a higher pressure. The rate of the reaction was monitored by the drop in pressure in this vessel. After the consumption of ethylene had essentially stopped (71 minutes) the autoclave was cooled rapidly in an ice bath. The autoclave was vented, opened, and the contents removed and analyzed by gas chromatography. Conversion of butadiene was 97%. Selectivity to *cis*-1,4-hexadiene was 99.9%. The only other product of the reaction was 2,4-hexadiene. Vinylcyclohexene, which is an impurity in the butadiene, was also found. The observed first-order rate constant for the reaction was 0.106 min⁻¹. The rate constant per mmole Co is 10.6

### Example 2

This example illustrates that the rate of the reaction doubles when the amount of starting cobalt complex is doubled. The reaction was carried out in a similar manner and at the same temperature and pressure as in Example 1.

11 mg (0.02 mmole) of the preformed complex of CoC1₂(1,3- bis (diphenylphosphino) propane) was dissolved in 80 mL anhydrous toluene. To this was added with stirring a 25 wt% solution of diethylaluminum chloride in toluene (0.6 mL, 1.0 mmole). Only 11.8 g of butadiene was added to the autoclave, and consumption of ethylene stopped after 37 minutes. Conversion of butadiene was 94%. Selectivity to cis-1,4-hexadiene was 100%. A trace of 2,4-hexadiene (less than 0.1%) was found. The observed first-order rate constant for the reaction was 0.206 min⁻¹. The rate constant per mmole Co is 10.3.

### Example 3

This example illustrates the use of two equivalents of triethylaluminum per cobalt in addition to an excess of diethylaluminum chloride.

The reaction was carried out in a similar manner and at the same temperature and pressure as Example 1. The preformed complex CoC1₂ (1,3-bis (diphenylphosphino) propane) (11 mg, 0.02 mmole) was dissolved in 80 mL anhydrous toluene. To this was added with stirring a 25 wt% solution of diethylaluminum chloride in toluene (0.5 mL, 0.96 mmole) and also a 2.5 wt% solution of triethylaluminum in toluene (0.2 mL, 0.04 mmole). Butadiene (22.7 g) was added to the autoclave, and consumption of ethylene stopped after 25 minutes. Conversion of butadiene was 98%. Selectivity to *cis*-1,4-hexadiene was 100%. A trace of 2,4-hexadiene (less than 0.1%) was found. The observed first-order rate constant for the reaction was 0.206 min⁻¹. The rate constant per mmole Co is 10.3.

### Tables 1, 2 and 3

The data of Examples 1, 2 and 3 for *cis*-1,4-hexadiene production using a preformed cobalt chloride-dppp catalyst and DEAC cocatalyst at a temperature less than 70° C are set forth in Table 1, arranged in descending order of overall rate of reaction. The surprising difference between these results and those using the same catalyst but with a TEAL cocatalyst at temperatures above 70° C are seen by comparison of the data of Table 1 with the data of following Table 2, a tabulation of data from examples contained in US-A-3,405,194, also arranged in the same descending order. There is an order of magnitude greater rate of reaction when using a preformed cobalt chloride-dppp catalyst and DEAC cocatalyst at less than 70° C in accordance with the invention, compared to using a preformed cobalt chloride-dppp catalyst and TEAL cocatalyst at more than 70° C; further, the selectivity to 1,4-hexadiene is essentially 100% when using the preformed cobalt chloride-dppp catalyst and DEAC cocatalyst at less than 70° C, but is less than 90% with the prior art catalyst system at the higher temperatures. The same improvement exists whether the prior art process CoC1₂-dppp is preformed or not. Table 3 tabulates data calculated from examples in the US-A-3,405,194 patent in which the CoC1₂-dppp catalyst was formed *in situ* and used in the presence of TEAL at temperatures above 70° C. Reaction rates are on the same order as the preformed catalyst set out in Table 2. In Tables 1,2 and 3, "Al" is aluminum, "Co" is cobalt, "Cmpd" is compound, "BD" is butadiene, "Di" is diene, and "1,4-HD" is 1,4-hexadiene. "Prodt'y" is catalyst productivity. "Conv" is conversion of butadiene to 1,4-hexadiene, and "Select'y" is selectivity of conversion of butadiene to 1,4-hexadiene. "TMAL" is Trimethyl Aluminum.

**Table 1**

| **Preformed CoC1**_{**2**}**(dppp) Catalyst, DEAC Cocatalyst Less Than 70° C** | | | |
|---|---|---|---|
| Ex | 3 | 1 | 2 |
| Co Cmpd | CoC1₂ (dppp) | CoC1₂ (dppp) | CoC1₂ (dppp) |
| Organo-Aluminum Cmpd | DEAC+ TEAL | DEAC | DEAC |
| A1 (mmol) | 1.00 | 1.00 | 1.00 |
| A1/Co | 50 | 100 | 50 |
| Solvent | toluene | toluene | toluene |
| BD (gms) | 22.70 | 20.80 | 11.80 |
| BD (mmol) | 420 | 385 | 219 |
| Di/Co | 21,019 | 38,519 | 10,926 |
| Time (min) | 25 | 71 | 37 |
| Temp (°C) | 60 | 60 | 60 |
| 1,4-HD Yield (gms) | 33.78 | 30.61 | 16.84 |
| 1,4-HD Yield (mols) | 0.41 | 0.37 | 0.21 |
| Prodt'y (mol/mol) | 20,563 | 37,262 | 10,253 |
| Rate (mol/mol/min) | 823 | 525 | 277 |
| Observed 1st Order Rate K (min⁻¹) | 0.206 | 0.106 | 0.206 |
| Rate per mmol Co | 10.30 | 10.60 | 10.30 |
| Conv (%) | 98 | 97 | 94 |
| Select'y (%) | 100 | 99.9 | 100 |

**Table 2**

| **U.S. Patent 3,405,194: Preformed CoC1**_{**2**}**(dppp) Catalyst TEAL Cocatalyst, More Than 70° C** | | | | | | | |
|---|---|---|---|---|---|---|---|
| Ex. | 23 | 24 | 21 | 22 | 20 | 19 | 18 |
| Co Cmpd | CoC1₂ (dppp) | CoC1₂ (dppp) | CoC1₂ (dppp) | CoC1₂ (dppp) | CoC1₂ (dppp) | CoC1₂ (dppp) | CoC1₂ (dppp) |
| Co (mmol) | 0.25 | 0.18 | 0.25 | 0.13 | 0.50 | 0.50 | 0.50 |
| Organo-Aluminum Cmpd | TMAL | TEAL | TEAL | TEAL | TEAL | TEAL | TEAL |
| A1 (mmol) | 15.65 | 8.47 | 10.95 | 10.95 | 1.46 | 5.11 | 5.11 |
| A1/Co | 63 | 46 | 44 | 88 | 3 | 10 | 10 |
| Solvent | toluene | ethylene chloride | toluene | toluene | toluene | toluene | toluene |
| BD (gms) | 114 | 85.7 | 100 | 100 | 100 | 20.5 | 20.5 |
| BD (mmol) | 2,111 | 1,587 | 1,852 | 1,852 | 1,852 | 380 | 380 |
| Di/Co | 8,444 | 8,625 | 7,4017 | 14,815 | 3,704 | 759 | 759 |
| Time (min) | 180 | 120 | 180 | 180 | 156 | 60 | 1140 |
| Temp (° C) | 80-90 | 98-102 | 80-90 | 80-90 | 80-90 | 80-90 | 80-90 |
| 1,4-HD Yield (g) | 161.80 | 67.20 | 116.70 | 51.80 | 105.80 | 24.40 | 13.40 |
| 1,4-HD Yield (mols) | 1.97 | 0.82 | 1.42 | 0.63 | 1.29 | 0.30 | 0.16 |
| Prodt'y (mol/mol) | 7,879 | 4,446 | 5,683 | 5,045 | 2,576 | 594 | 326 |
| Rate (mol/mol/min) | 43.77 | 37.05 | 31.57 | 28.03 | 16.51 | 9.90 | 0.29 |
| Conv (%) | 107.41 | 65.05 | 89.25 | 40.41 | 93.87 | 112.59 | 149.37 |
| Select'y (%) | 87.01 | 79.37 | 86.11 | 84.41 | 74.22 | 69.61 | 28.82 |

**Table 3**

| **U.S. Patent 3,405,194: In Situ CoC1**_{**2**}**(dppp) Catalyst TEAL Cocatalyst, More Than 70° C** | | | |
|---|---|---|---|
| Ex. | 27 | 26 | 25 |
| Co Cmpd | CoC1₂+ dppp | CoC1₂+ 0.5 dppp | CoC1₂+ dppp |
| Co (mmol) | 0.13 | 0.25 | 0.50 |
| Organo-Aluminum Cmpd | TEAL | TEAL | TEAL |
| A1 (mmol) | 7.27 | 7.30 | 7.30 |
| A1/Co | 58 | 29 | 15 |
| Solvent | ethylene chloride | chlorobenzene | chlorobenzene |
| BD (gms) | 67 | 67 | 67 |
| BD (mmol) | 1,241 | 1,241 | 1,241 |
| Di/Co | 9,926 | 4,963 | 2,481 |
| Time (min) | 180 | 180 | 180 |
| Temp (° C) | 80-90 | 80-90 | 80 |
| 1,4-HD Yield (g) | 83.70 | 78.50 | 85.00 |
| 1,4-HD Yield (mols) | 1.02 | 0.96 | 1.04 |
| Prodt'y (mol/mol) | 8,151 | 3,822 | 2,069 |
| Rate (mol/mol/min) | 45.28 | 21.23 | 17.24 |
| Conv (%) | 85.75 | 92.72 | 96.80 |
| Select'y (%) | 95.93 | 83.21 | 86.30 |

**Comparative Example 4 and Example 5** which follow demonstrate that use of excess triethylaluminum (TEAL) as cocatalyst at less than 70° C yields unsatisfactory results with the preformed cobalt chloride-dppp catalyst system of the present invention compared to the use of excess diethylaluminum chloride (DEAC) as cocatalyst. In these two examples the preformed complex CoC1₂(dppp) is initially dissolved in a small amount of chlorobenzene in order to assure it is completely dissolved before addition of the cocatalyst.

### Comparative Example 4

The reaction was carried out in a similar manner and at the same temperature and pressure as in Example 1. The preformed complex CoC1₂(dppp) (27 mg, 0.05 mmole) was dissolved in 10 mL anhydrous chlorobenzene. To this was added with stirring a 25 wt% solution of triethylaluminum (TEAL) in toluene (0.8 mL, 1.5 mmole). Anhydrous toluene (70 mL) was then added to this solution. Butadiene (21.6 g) was added to the autoclave at 60° C, which was pressured with ethylene, and the reaction was allowed to proceed for 14 minutes. No significant uptake of ethylene was observed during this time. Conversion of butadiene to *cis*-1,4-hexadiene was less than 1%. No other product of the reaction was observed.

### Example 5

The reaction was carried out in a similar manner and at the same temperature and pressure as in Example 1. The preformed complex CoC1₂(dppp) (27 mg, 0.05 mmole) was dissolved in 10 mL anhydrous chlorobenzene. To this was added with stirring a 25 wt% solution of diethylaluminum chloride (DEAC) in toluene (0.8 mL, 1.5 mmole). Anhydrous toluene (70 mL) was then added to this solution. Butadiene (21.1 g) was added to the autoclave at 60° C, which was pressured with ethylene, and the reaction was allowed to proceed for 30 minutes. During the first few minutes the reaction rate was so fast that the temperature could not be controlled. Consumption of ethylene had essentially ceased after 6 minutes. Conversion of butadiene was greater than 99%. Selectivity to *cis*-1,4-hexadiene was 99.9%; the only other product of the reaction was 2,4-hexadiene.

### Example 6

This Example illustrates the use of Co(acac)₃ as the starting cobalt compound component of the complex. Twice as much of this cobalt compound is needed to achieve essentially the same observed first-order rate constant as with the preformed complex.

The reaction was carried out in a similar manner and at the same temperature and pressure as Example 1. Cobalt(**III**) acetylacetonate (14 mg, 0.04 mmole), and 1,3-bis (diphenylphosphino) propane (19 mg, 0.045 mmole) were dissolved in 80 mL anhydrous toluene. To this was added with stirring a 25 wt% solution of diethylaluminum chloride in toluene (0.6 mL, 1.0 mmole). Butadiene (24.0 g) was added to the autoclave, which was pressured with ethylene, and consumption of ethylene stopped after 27 minutes. Conversion of butadiene was 99%. Selectivity to *cis*-1,4-hexadiene was 99.9%. The only other product of the reaction was 2,4-hexadiene. The observed first-order rate constant for the reaction was 0.195 min⁻¹. The rate constant per mmole Co is 4.9.

### Example 7

This Example illustrates the use of Co(acac)₂ as the starting cobalt compound component of the complex. Use of this cobalt compound in the same relative molar amounts results in a rate of reaction about one and a half times that found when the preformed complex CoC1₂-dppp is used (as in Example 1).

The reaction was carried out in a similar manner and at the same temperature and pressure as in Example 1. Cobalt(**II**) acetylacetonate (2.6 mg, 0.01 mmole) and dppp (6.2 mg, 0.015 mmole, dppp = 1,3-bis (diphenylphosphino) propane) were dissolved in 80 mL anhydrous toluene. This was accomplished by using 2 mL of a stock solution made by dissolving Co(acac)₂ (26 mg, 0.10 mmole) and dppp (62 mg, 0.15 mmole) in 20 mL of anhydrous toluene. To the 80 mL catalyst solution was added with stirring a 25 wt% solution of DEAC in toluene (0.6 mL, 1.0 mmole). Butadiene (22.5 g) was added to the autoclave at 60° C, which was pressured with ethylene, and consumption of ethylene stopped after 32 minutes. Conversion of butadiene was 98%. Selectivity to *cis*-1,4-hexadiene was 100%; a trace of 2,4-hexadiene (less than 0.1%) was found. The observed first-order rate constant for the reaction was 0.150 min⁻¹. The rate constant per mmole Co is 15.00.

### Example 8

This Example illustrates the use of the product *cis*-1,4-hexadiene as solvent for the reaction.

The reaction was carried out in a similar manner and at the same temperature and pressure as in Example 1. Cobalt(**II**) acetylacetonate (10 mg, 0.04 mole) and dppp (19 mg, 0.045 mmole) were dissolved in 80 mL *cis*-1,4-hexadiene (dried by passing through a column of 3A molecular sieve and alumina under nitrogen). To this was added with stirring a 25 wt% solution of DEAC in toluene (0.6 mL, 1.0 mmole). Butadiene (22.9 g) was added to the autoclave at 60° C, which was pressured with ethylene, and consumption of ethylene stopped after 23 minutes. Conversion of butadiene was 99%. Selectivity to *cis*-1,4-hexadiene (based on product formed in the reaction) was 99.9%; the only other product of the reaction was 2,4-hexadiene. The observed first-order rate constant for the reaction was 0.095 min^{**-**}¹. The rate constant per mmole Co is 2.38.

### Example 9

This example illustrates the use of isoprene as the conjugated diene reactant. In the reaction of isoprene with ethylene, the product is a mixture of 4-methyl-1,4-hexadiene and 5-methyl-1,4-hexadiene.

The reaction was carried out in a similar manner and at the same temperature and pressure as in Example 1. Cobalt(**II**) acetylacetonate (31 mg, 0.12 mmole) and dppp (52 mg, 0.125 mmole) were dissolved in 80 mL anhydrous ortho-xylene. To this was added with stirring at 25 wt% solution of DEAC in toluene (3.3 mL, 6 mole). Isoprene (23.6 g, dried by passing through a column of 3A molecular sieve and alumina) was added to the autoclave at 60° C, which was pressured with ethylene, and consumption of ethylene stopped after 33 minutes. Conversion of isoprene was 99%. Selectivity to 4-methyl-1,4-hexadiene (88%) and 5-methyl-1,4-hexadiene (12%) was essentially 100%. The observed first-order rate constant for the reaction was 0.218 min⁻¹. The rate constant per mmole Co is 1.82.

### Example 10

The Example shows that using a molar ratio of dppp ligand to Co(acac)ₙ that is less than two is preferred. In this example, even though the amount of the cobalt salt of highest activity was quadrupled and a 2:1 ratio of dppp ligand to Co(acac)₂ was used, a much slower rate of reaction resulted. A molar ratio of dppp ligand of from a little less than unity to less than 2 is preferred, as in Example 7, which uses a dppp:Co ratio of 1.5:1, yet achieves significantly greater activity with one-fourth the amount of cobalt.

The reaction was carried out in a similar manner and at the same temperature and pressure as in Example 7 but with a 2:1 dppp:Co mol ratio. Cobalt(**II**) acetylacetonate (10 mg, 0.04 mmole) and dppp (33 mg, 0.08 mmole) were dissolved in 80 mL anhydrous toluene. To this was added with stirring a 25 wt% solution of DEAC in toluene (0.6 mL, 1.0 mmole). Butadiene (20.3 g) was added to the autoclave at 60° C, which was pressured with ethylene, and the reaction was allowed to proceed for 60 minutes. At this time conversion of butadiene was only 76%. Selectivity to *cis*-1,4-hexadiene was 99.9%; the only other product of the reaction was 2,4-hexadiene. The observed first-order rate constant for the reaction was 0.040 min⁻¹. The rate constant per mmole Co is 1.00.

### Tables 4 and 5.

Table 4 sets forth the data of Examples 6 - 10, in descending order of rate of reaction. The rate of reaction for butadiene to 1,4-hexadiene is at least about 120 (119) mols of 1,4-hexadiene per mol of cobalt catalyst pre minute. When less than 2 mols of dppp are used per mol of Co(acac)₂ or Co(acac)₃, rates of reaction are from about 4 (Examples 6,8) to about 11 (Example 7) times higher than with 2 mols of dppp per mol of Co(acac)₂ or Co(acac)₃. In all instances, selectivity to 1,4-hexadiene is essentially 100%.

Table 5 tabulates data from the comparable examples of US-A-3,405,194 (these use a Co(acac)₃ combined with dppp catalyst). In Example 43 of the patent, a DEAC cocatalyst is used at a temperature above 70° C. Rate of reaction was very slow, about one fifth that of the minimum preferred rate for the Co(acac)ₙ combined with dppp system at less than 70° C. In patent Example 88, the monoolefin is propylene and the rate is about a third that of Example 43 of the patent.

In the tables, "IP" is isoprene, "C2" is ethylene, "C3" is propylene, and other abbreviations are the same as in Tables 1, 2 and 3.

**Table 4**

| **Co(acac)**_{**n**} **Combined With dppp Catalyst, DEAC Cocatalyst Less Than 70° C** | | | | | |
|---|---|---|---|---|---|
| Ex. | 7 | 8 | 6 | 10 | 9 |
| Co Cmpd | Co(acac)₂ + 1.5 dppp | Co(acac)₂ + dppp | Co(acac)₃ + dppp | Co(acac)₂ + 2 dppp | Co(acac)₂ + dppp |
| Co (mmol) | 0.01 | 0.04 | 0.04 | 0.04 | 0.12 |
| Organo-Aluminum Cmpd | DEAC | DEAC | DEAC | DEAC | DEAC |
| A1 (mmol) | 1.00 | 1.00 | 1.00 | 1.00 | 6.00 |
| A1/Co | 100 | 25 | 25 | 25 | 50 |
| Solvent | toluene | 1,4-HD | toluene | toluene | o-xylene |
| Di | BD | BD | BD | BD | IP |
| Di (gms) | 22.5 | 22.9 | 24.00 | 20.3 | 23.6 |
| Di (mmol) | 417 | 424 | 444 | 376 | 350 |
| Di/Co | 41,667 | 10,602 | 11,111 | 9,398 | 2,917 |
| C= | C2 | C2 | C2 | C2 | C2 |
| Time (min) | 32 | 23 | 27 | 60 | 33 |
| Temp (° C) | 60 | 60 | 60 | 60 | 60 |
| 1,4-HD Yield (g) | 33.48 | 34.39 | 36.04 | 23.40 | 32.99 |
| 1,4-HD Yield (mols) | 0.41 | 0.42 | 0.44 | 0.29 | 0.34 |
| Prodt'y (mol/mol) | 40,764 | 10,468 | 10,970 | 7,123 | 2,858 |
| Rate (mol/mol /min) | 1,274 | 455 | 406 | 119 | 87 |
| Observed 1st Order Rate K (min⁻¹) | 0.15 | 0.095 | 0.195 | 0.04 | 0.218 |
| Rate/mmol | 15.00 | 2.38 | 4.90 | 1.00 | 1.82 |
| Conv (%) | 98 | 99 | 99 | 76 | 99 |
| Select'y (%) | 100 | 99.9 | 99.9 | 99.9 | 100 |

**Table 5**

| **U.S. Patent 3,405,194; Co(acac)**_{**3**} **Combined With dppp Catalyst Organoaluminum Cocatalyst, More Than 70° C** | | |
|---|---|---|
| Ex. | 43 | 88 |
| Co Cmpd | Co(acac)₃ + dppp | Co(acac)₃ + 2 dppp |
| Co (mmol) | 0.50 | 0.10 |
| Organo-Aluminum Cmpd | DEAC | TEAL |
| A1 (mmol) | 8.0 | 7.3 |
| A1/Co | 16 | 73 |
| Solvent | toluene | ethylene chloride |
| Di | BD | BD |
| BD (gms) | 16.2 | 21.1 |
| BD (mmol) | 0.31 | 0.39 |
| Di/Co | 620 | 3900 |
| C= | C2 | C3 |
| Time (min) | 25 | 300 |
| Temp (° C) | 80 | 90-100 |
| 1,4-HD Yield (g) | 23.70 | 22.10 |
| 1,4-HD Yield (mols) | 0.29 | 0.23 |
| Prodt'y (mol/mol) | 577 | 2298 |
| Rate (mol/mol/min) | 23.08 | 7.66 |
| Conv (%) | 97.96 | 79.33 |
| Select'y (%) | 98.34 | 74.26 |

In the following Examples 11 through 14, results of using a catalyst system having a 1,2-bis (diphenylphosphino)ethane (dppe) catalyst ligand at a temperature less than 70° C are shown.

Comparative Examples 11 and 12 show that a substantially slower rate of reaction results when dppe is combined with Co(acac)₂ and DEAC at a temperature of 60° C. This is the case with a molar ratio of dppe:Co of either 1:1 or 2:1.

### Comparative Example 11

The reaction was carried out in a similar manner and at the same temperature and pressure as in Example 1. Cobalt(**II**) acetylacetonate (10 mg, 0.04 mmole) and dppe (16 mg, 0.04 mmole) were dissolved in 80 mL anhydrous toluene. To this was added with stirring a 25 wt% solution of diethylaluminum chloride in toluene (0.6 mL, 1.0 mmole). Butadiene (22.3 g) was added to the autoclave at 60° C, which was pressured with ethylene, and the reaction was allowed to proceed for 60 minutes. Conversion of butadiene was 99%. Selectivity to *cis*-1,4-hexadiene was 99.4%; the only other product of the reaction was 2,4-hexadiene. The observed first order rate constant for the reaction was 0.073 min⁻¹. (Rate constant) / (mmole Co) = 1.83.

### Comparative Example 12

The reaction was carried out in a similar manner and at the same temperature and pressure as in Example 1. (Cobalt(**II**) acetylacetonate (10 mg, 0.04 mmole) and dppe (32 mg, 0.08 mmole) were dissolved in 80 mL anhydrous toluene. To this was added with stirring a 25 wt% solution of diethylaluminum chloride in toluene (0.6 mL, 1.0 mmole). Butadiene (20.6 g) was added to the autoclave at 60° C, which was pressured with ethylene, and the reaction was allowed to proceed for 60 minutes. Conversion of butadiene was 99%. Selectivity to *cis*-1,4-hexadiene was 99.6%; the only other product of the reaction was 2,4-hexadiene. The observed first-order rate constant for the reaction was 0.116 min⁻¹. (Rate constant) / (mmole Co) = 2.90.

Comparative Examples 13 and 14 which follow show that a substantially slower rate of reaction results when dppe is combined with Co(acac)₃ and DEAC at a temperature less than 70° C. This is the case with a dppe:Co molar ratio of either 1:1 or 2:1.

### Comparative Example 13

The reaction was carried out in a similar manner and at the same temperature and pressure as in Example 1. Cobalt(**III**) acetylacetonate (14 mg, 0.04 mmole) and dppe (16 mg, 0.04 mmole) were dissolved in 80 mL anhydrous toluene. To this was added with stirring a 25 wt% solution of DEAC in toluene (0.6 mL, 1.0 mmole). Butadiene (20.1 g) was added to the autoclave at 60° C, which was pressured with ethylene, and the reaction was allowed to proceed for 60 minutes. Conversion of butadiene was 99%. Selectivity to *cis*-1,4-hexadiene was 99.6%; the only other product of the reaction was 2,4-hexadiene. The observed first order rate constant for the reaction was 0.114 min⁻¹. (Rate constant) / (mmole Co) = 2.85.

### Comparative Example 14

The reaction was carried out in a similar manner and at the same temperature and pressure as in Example 1. Cobalt(**III**) acetylacetonate (14 mg, 0.04 mmole) and dppe (32 mg, 0.08 mmole) were dissolved in 80 mL anhydrous toluene. To this was added with stirring a 25 wt% solution of DEAC in toluene (0.6 mL, 1.0 mmole). Butadiene (23.6 g) was added to the autoclave at 60° C, which was pressured with ethylene, and the reaction was allowed to proceed for 49 minutes. Conversion of butadiene was 76%. Selectivity to cis-1,4-hexadiene was 99.75; the only other product of the reaction was 2,4-hexadiene. The observed first order rate constant for the reaction was 0.020 min⁻¹. (Rate constant) / (mmole Co) = 0.50.

### Tables 6 and 7

The data of Examples 11, 12 13 and 14, all obtained at a reaction temperature less than 70° C, are set forth in Table 6, in descending order of rate of reaction. The following Table 7 sets forth tabulations of data calculated from the patent examples of US-A-3,405,194 obtained at a reaction temperature less than 70° C. The quantities of Co(acac)ₙ employed in Examples 11 through 14 are the same as the quantities employed in Example 6 for Co(acac)₃ combined with dppp (toluene solvent) and Example 8 for Co(acac)₂ combined with dppp (1,4-hexadiene solvent). As seen from Table 6, a catalyst system employing the ligand 1,2-bis (diphenylphosphino) ethane, or "dppe" as abbreviated herein, in combination with Co(acac)₂ or Co(acac)₃, even in the presence of a dialkylaluminum halide, and even at the same temperatures as used in Examples 6 and 8, is less than half as catalytically active as Examples 6 and 8. The reaction rates for the comparable patent examples shown in table 7 show an extremely slow reaction rate, more than 30 times slower than the rates of Examples 11 through 14. In Table 7, "EASC" is ethyl aluminum sesquichloride.

**Table 6**

| **Co(acac)**_{**n**}**Combined With dppe Catalyst DEAC Cocatalyst, Less Than 70° C** | | | | |
|---|---|---|---|---|
| Ex. | 11 | 14 | 12 | 13 |
| Co Cmpd | Co(acac)₂ + dppe | Co(acac)₃ + dppe | Co(acac)₂ + 2 dppe | Co(acac)₃ + dppe |
| Co (mmol) | 0.01 | 0.01 | 0.04 | 0.04 |
| Organo-Aluminum Cmpd | DEAC | DEAC | DEAC | DEAC |
| A1 (mmol) | 1.00 | 1.00 | 1.00 | 1.00 |
| A1/Co | 25 | 25 | 25 | 25 |
| Solvent | toluene | toluene | toluene | toluene |
| BD (gms) | 22.3 | 23.6 | 20.6 | 20.1 |
| BD (mmol) | 413 | 437 | 381 | 372 |
| Di/Co | 10,324 | 10,926 | 9,537 | 9,306 |
| Time (min) | 60 | 49 | 60 | 60 |
| Temp (° C) | 60 | 60 | 60 | 60 |
| C= | C2 | C2 | C2 | C2 |
| 1,4-HD Yield (g) | 33.32 | 27.15 | 30.84 | 30.10 |
| 1,4-HD Yield (mols) | 0.41 | 0.33 | 0.38 | 0.37 |
| Prodt'y (mol/mol) | 10,142 | 8,265 | 9,388 | 9,160 |
| Rate (mol/mol/min) | 169 | 169 | 156 | 153 |
| Observed 1st Order Rate K (min⁻¹) | 0.073 | 0.02 | 0.116 | 0.114 |
| Rate/mmol | 1.83 | 0.50 | 2.90 | 2.85 |
| Conv (%) | 99 | 76 | 99 | 99 |
| Select'y (%) | 99.4 | 99.7 | 99.6 | 99.6 |

**Table 7**

| **U.S. Patent 3,405,194: Co(acac)**_{**n**} **Combined With dppp Catalyst Organoaluminum Cocatalyst, Less Than 70° C** | | |
|---|---|---|
| Ex. | 39 | 47 |
| Co Cmpd | Co(acac)₃ + 2.2 dppp | Co(acac)₃ + 2.2 dppp |
| Co (mmol) | 1.00 | 1.00 |
| Organo-Aluminum Cmpd | DEAC | EASC |
| A1 (mmol) | 15.9 | 17.6 |
| A1/Co | 15.9 | 17.6 |
| Solvent | ethane | toluene |
| BD (gms) | 32.4 | 16.7 |
| BD (mmol) | 0.60 | 0.31 |
| Di/Co | 600 | 310 |
| Time (min) | 120 | 1380 |
| Temp (° C) | 50 | 50 |
| C= | C2 | C2 |
| 1,4-HD Yield (g) | 42.50 | 11.30 |
| 1,4-HD Yield (mols) | 0.52 | 0.14 |
| Prodt'y (mol/mol) | 517 | 138 |
| Rate (mol/mol/min) | 4.31 | 0.10 |
| Conv (%) | 91.31 | 47.71 |
| Select'y (%) | 94.60 | 93.39 |

## Claims

1. A process for producing substantially pure 1,4 dienes by converting a conjugated diolefin to the 1,4-diene with substantially 100 % selectivity, which comprises reacting a conjugated diolefin with an alpha-olefin in a hydrocarbon solvent reaction medium and in the presence of a catalyst system consisting essentially of a cobalt compound, a tertiary diphosphine and an organo aluminum compound, characterized in that the catalyst system comprises:
a) Co(acac)ₙ wherein from 0.9 to less than 2 mols of dppp are combined with the Co(acac)ₙ, or a preformed complex of CoCl₂(dppp), where acac is acetylacetonate, dppp is 1,3-bis (diphenylphosphino)propane, and n is the valence of cobalt; and
b) a dialkylaluminum chloride,
wherein the catalyst system has an Al/Co molar ratio of at least 5:1, the reaction is performed at a temperature of at least 50°C and less than 70°C and at an alpha-olefin partial pressure of from 50 to 10,000 kPa (0.5 to 100 atmospheres)

2. The process of claim 1, wherein the hydrocarbon solvent is non-halogenated.

3. The process of claim 2, wherein the solvent is toluene, xylene, a 1,4-diene which is the product of the reaction, or a mixture of any two or more thereof.

4. The process of any preceding claim, wherein the conjugated diolefin is butadiene or isoprene, the alpha-olefin is ethylene or propylene and the 1,4-diene is 1,4-hexadiene, 2-methyl-1,4-hexadiene, or a mixture of 4-methyl-1,4-hexadiene and 5-methyl-1,4-hexadiene.

5. The process of any preceding claim, wherein the dialkylaluminum chloride is diethylaluminum chloride.

6. The process of any preceding claim wherein the catalyst system comprises CoCl₂(dppp) and further includes triethylaluminum with no more than two molar equivalents of triethylaluminum per mol CoCl₂(dppp).

## Patentansprüche

1. Verfahren zur Herstellung von im Wesentlichen reinen 1,4-Dienen durch Umwandlung eines konjugierten Diolefins zum 1,4-Dien mit im Wesentlichen 100 % Selektivität, bei dem ein konjugiertes Diolefin mit einem α-Olefin in einem Kohlenwasserstoff-Lösungsmittel als Reaktionsmedium und in Gegenwart eines Katalysatorsystems umgesetzt wird, das im Wesentlichen aus einer Kobaltverbindung, einem tert. Diphosphin und einer Organoaluminiumverbindung besteht, dadurch gekennzeichnet, dass das Katalysatorsystem umfasst:
a) Co(acac)ₙ, wobei 0,9 bis weniger als 2 mol dppp mit dem Co(acac)ₙ kombiniert sind, oder einen vorgeformten Komplex CoCl₂(dppp), wobei acac Acetylacetonat, dppp 1,3-Bis(diphenylphosphino)propan und n die Wertigkeit des Kobalts ist, und
b) ein Dialkylaluminiumchlorid,
wobei das Katalysatorsystem ein molares Verhältnis Al/Co von mindestens 5 : 1 aufweist und die Reaktion bei einer Temperatur von mindestens 50 °C und weniger als 70 °C und bei einem Partialdruck des α-Olefins von 50 bis 10 000 kPa (0,5 bis 100 Atmosphären) durchgeführt wird.

2. Verfahren nach Anspruch 1, bei dem das Kohlenwasserstoff-Lösungsmittel nicht halogeniert ist.

3. Verfahren nach Anspruch 2, bei dem das Lösungsmittel Toluol, Xylol, ein 1,4-Dien, welches das Reaktionsprodukt ist, oder ein Gemisch von zwei oder mehr derselben ist.

4. Verfahren nach einem der vorangehenden Ansprüche, bei dem das konjugierte Diolefin Butadien oder Isopren, das α-Olefin Ethylen oder Propylen und das 1,4-Dien 1,4-Hexadien, 2-Methyl-1,4-hexadien oder ein Gemisch von 4-Methyl-1,4-hexadien und 5-Methyl-1,4-hexadien ist.

5. Verfahren nach einem der vorangehenden Ansprüche, bei dem das Dialkylaluminiumchlorid Diethylaluminiumchlorid ist.

6. Verfahren nach einem der vorangehenden Ansprüche, bei dem das Katalysatorsystem CoCl₂(dppp) umfasst und außerdem Triethylaluminium in einer Menge von nicht mehr als zwei molaren Äquivalenten Triethylaluminium pro mol CoCl₂(dppp) enthält.

## Revendications

1. Procédé pour la production de 1,4-diènes pratiquement purs par conversion d'une dioléfine conjuguée en le 1,4-diène avec une sélectivité pratiquement égale à 100 %, qui comprend la réaction d'une dioléfine conjuguée avec une alpha-oléfine dans un milieu réactionnel consistant en un solvant hydrocarboné et en présence d'une formulation de catalyseur consistant essentiellement en un composé de cobalt, une diphosphine tertiaire et un composé organique d'aluminium, caractérisé en ce que la formulation de catalyseur comprend :
a) du Co(acac)ₙ, dans lequel une quantité de 0,9 à moins de 2 moles de dppp est combinée au Co(acac)ₙ, ou un complexe préformé de CoCl₂(dppp), le terme "acac" signifiant acétylacétonate, le terme "dppp" désignant le 1,3-bis-(diphénylphosphino)propane et n représentant la valence du cobalt ; et
b) un chlorure de dialkylaluminium,
dans lequel la formulation de catalyseur a un rapport molaire de Al/Co d'au moins 5:1, la réaction est conduite à une température d'au moins 50°C et inférieure à 70°C et à une pression partielle d'alpha-oléfine comprise dans l'intervalle de 50 à 10 000 kPa (0,5 à 100 atmosphères).

2. Procédé suivant la revendication 1, dans lequel le solvant hydrocarboné est non halogéné.

3. Procédé suivant la revendication 2, dans lequel le solvant consiste en toluène, xylène, un 1,4-diène qui est le produit de la réaction ou un mélange de deux ou plus de deux quelconques de ces solvants.

4. Procédé suivant l'une quelconque des revendications précédentes, dans lequel la dioléfine conjuguée consiste en butadiène ou isoprène, l'alphaoléfine consiste en éthylène ou propylène et le 1,4-diène consiste en 1,4-hexadiène, 2-méthyl-1,4-hexadiène ou un mélange de 4-méthyl-1,4-hexadiène et de 5-méthyl-1,4-hexadiène.

5. Procédé suivant l'une quelconque des revendications précédentes, dans lequel le chlorure de dialkylaluminium est le chlorure de diéthylaluminium.

6. Procédé suivant l'une quelconque des revendications précédentes, dans lequel la formulation de catalyseur comprend du CoCl₂(dppp) et comprend en outre du triéthylaluminium avec une quantité non supérieure à 2 équivalents molaires de triéthylaluminium par mole de CoCl₂(dppp).
